# EUROPEAN PATENT SPECIFICATION

(11) **EP 0 541 515 B1**
(45) Date of publication and mention of the grant of the patent: **03.05.1995**
(21) Application number: 93200022.7
(22) Date of filing: 16.03.1990
(51) Int. Cl.: A61M 39/04

(54) **Pre-slit injection site usable with a blunt cannula**
Vorgeschlitzte Injektionsstelle sowie konische Kanüle
Site d'injection pré-fendue avec cannule émoussée

(30) Priority: 17.03.1989 US 325617
(43) Date of publication of application: 12.05.1993
(62) Divisional of application: 90905119.5
(73) Proprietor: BAXTER INTERNATIONAL INC., Deerfield, Illinois 60015 (US)
(72) Inventor: Jepson, Steven C., Palatine, Illinois 60067 (US); Dudar, Thomas E., Palatine, Illinois 60067 (US); Finley, Michael J., Park City, Illinois 60085 (US); Desecki, Vincent C., Ingleside, Illinois 60041 (US)
(74) Representative: MacGregor, Gordon

(56) References cited:
- EP-A- 0 114 677
- WO-A-89/06553
- DE-A- 2 805 354
- DE-C- 3 303 718
- DE-U- 8 425 197
- FR-A- 2 049 513

## Description

The present invention relates to an injection or sampling site usable with a blunt cannula device.

Injection sites usable with pointed cannulae have long been known, e.g. as disclosed in US-A-4412573. Injection sites usable with a blunt cannula device are also known, e.g. from US-A-4197848.

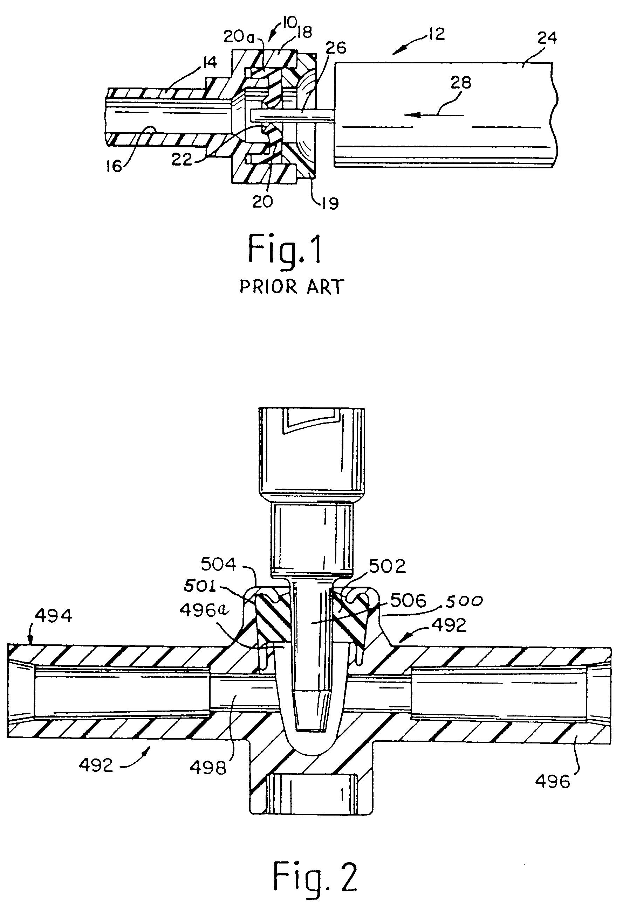

A prior art pre-slit injection site, for use with a blunt cannula device, is known having a cylindrical housing closed by a pre-slit septum, as illustrated in Fig. 1 of the accompanying drawings.

DE-U-8425197.2 discloses a pre-slit septum in a cylindrical housing for receiving a blunt cannula.

FR-A-2049513 discloses an in-line injection site of the general type with which the invention is concerned, as set out in the precharacterising part of Claim 1.

The present invention provides an in-line injection or sampling site having distinguishing features as set out in the characterising part of Claim 1.

The present invention provides an in-line injection or sampling site which can be used for the purpose of adding medication to a fluid stream or removing a sample from a fluid stream.

Reference is now made to the accompanying drawings, wherein:-
Fig.1 is a side elevational view, partly in section of a prior art pre-slit injection site and associated blunt-ended cannula device;
Fig.2 is a cross-sectional view of a pre-slit in-line injection or sampling site according to the invention, shown with an inserted blunt-ended cannula;
Fig. 3 is a perspective view of the injection or sampling site of Fig.2 in joined and locked relationship with an alternative blunt-ended cannula device;
Fig.4 is a perspective view, partially broken away, of a blunt-ended cannula device/syringe combination; and
Fig.5 is a perspective view of the cannula device/syringe of Fig.4 in joined relationship with the injection or sampling site of Fig.2

A prior art pre-slit injection site 10 and associated blunt cannula 12 are illustrated in Figure 1. The prior art injection site 10 has a cylindrical housing 14 with a fluid flow path 16 therethrough. A first end 18 of the housing 14 is closed with a relatively thin disc-shaped resealable member 20. The member 20 has a resealable opening 22 therein.

The member 20 is a molded septum with an integrally formed skirt 20a. The skirt 20a is oriented generally perpendicular to the portion of the septum with the opening 22.

The cannula 12 includes a body portion 24 which carries at a first end a hollow, cylindrical, blunt piercing member 26. As the cannula 12 is moved in a direction 28 toward the first end 18 of the injection site 10, the member 26 slidably engages the opening 22. The sealing member 20 is then deformed adjacent the opening 22 and the number 26 extends into the flow path 16. A fluid flow path through the cannula 12 will then be in fluid flow communication with the flow path 16 via the hollow piercing member 26.

Reference is made to WO-A-8906553 (EP-0354947) published 21 February 1990. This document teaches various constructions of pre-slit injection sites and blunt cannula devices as well as methods of mounting a septum. The disclosure of this document provides a full enabling disclosure for making pre-slit injection sites and blunt cannula devices and is incorporated in the present specification by reference, to avoid repetition of the matter disclosed therein.

Figure 2 shows, in cross-sectional view, an in-line slit injection or sampling site device 492, preferably for adding medication to a fluid stream, removing a sample from a fluid stream, or similar application. The device depicted in Figure 2 has a fluid entry port 494 at one end, a fluid exit port 496 at the other end, and a fluid passageway 498 communicating directly between the entry and exit ports. The inlet and outlet may have such additional features as are useful connecting the injection or sampling site device within a fluid flow path. As depicted, the inlet defines a slightly tapered female surface and the outlet defines a similarly female tapered surface which are preferably joined by solvent bonding a similar attachment to plastic tubing of an administration set, extension set or the like. Standard luer fittings or surfaces could also be provided at the inlet or outlet, as desired.

For injecting liquid into the fluid stream or sampling the fluid stream, the device has a side channel 496a which communicates between a pre-slit septum 502 and the fluid passageway 498. The side channel is formed in a housing 500 having an interior tapered surface 501. The septum 502 is received by the tapered surface, which applies radially directed forces to the sealing member. The taper is preferably 5° to 20° and may be 12°. The septum is made as described in EP-A-0354947, and mounted and held in position by a swaged-over wall 504 which may include a coloured identifier ring around the septum. The swaged over wall applies axial forces to the septum 502, thereby creating a domed exterior surface.

A blunt cannula, such as cannula 506, may be inserted through the pre-slit septum for injecting fluid into the liquid stream flowing between the inlet and outlet, or for taking samples of the fluid stream.

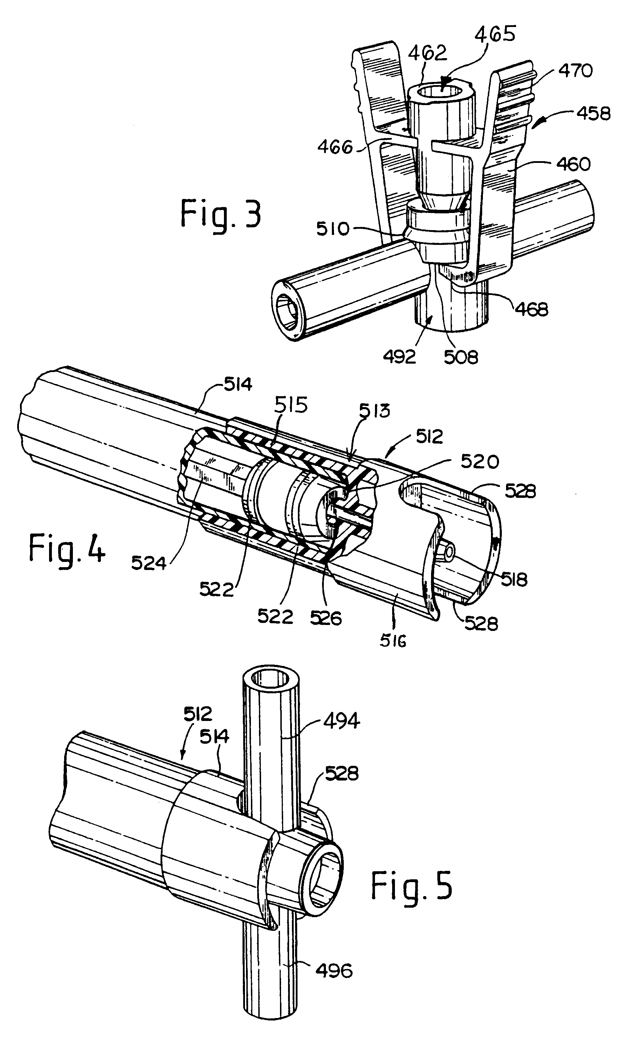

The in-line injection or sampling site device 492 shown in Figure 2 may be used in combination with a bare blunt cannula, such as that depicted in Figure 2, or may be used in combination with the blunt cannula device 458, depicted in Figure 3, when a locking relationship between the blunt cannula and injection or sampling site is desired. The device 458 utilises a pair of resilient gripping fingers 460 for retaining the blunt cannula on the injection or sampling site. The device 458 has a generally cylindrical, hollow base or body portion 462 and a blunt cannula portion (not shown). A fluid flow path extends through the blunt cannula portion and communicates with a female luer connection 465 defined in the hollow body portion for fluid flow through the blunt cannula device.

Each of the gripping fingers 460 is mounted to the body portion of the blunt cannula device by an intermediate radially extending wall portion 466. The gripping fingers have radially inwardly directed retention means 468 at one end and gripping means 470 at the other end for squeezing and spreading the retention means to release the blunt cannula device from the injection or sampling site. In the as-molded condition, the gripping fingers are biased radially inwardly, toward the blunt cannula portion 464. Because of the natural resilience of the plastic, the retention end of the fingers may be spread by squeezing the gripping end of the fingers. The natural resilience will hold the retention means in the lock position until manually released.

As depicted, the in-line injection or sampling site has a radially extending shoulder 508 on each side of the housing, for engaging against the retention means 468 on the end of the resilient gripping fingers 460. The in-line injection or sampling site also includes a generally tapered surface 510 defined on the exterior surface for spreading the retention means as the blunt cannula is inserted into the injection or sampling site. Insertion of the blunt cannula into the injection or sampling site results in the retention means being spread by the tapered surface 510 and, as the blunt cannula is inserted farther, the retention means snap into a locking position behind the radial shoulder 508. In this arrangement, the blunt cannula is securely locked onto the injection or sampling site and inadvertent withdrawal is thus prevented. To remove the blunt cannula from the in-line injection or sampling site, the gripping ends 470 of the resilient fingers are squeezed, causing spreading of the retention means 468 and release from the injection or sampling site. The cannula may then be simply removed by withdrawing it from the injection or sampling site.
Figure 4 depicts a blunt cannula device 512 in combination with a syringe 514. The blunt cannula device 512 has a generally cylindrical outer wall 513 defining a sheath 516 which encloses and substantially protects a blunt cannula portion 518. The blunt cannula portion is attached to and extends from an intermediate transverse interior wall 520. The blunt cannula device 512 may be attached to a syringe in various ways. As depicted, however, the syringe 514 has a glass barrel wall which is tightly press fit into one end portion 515 of the cylindrical outer wall, extending therewithin to the transverse wall 520.

Although various syringes may be used in connection with the blunt cannula device 512, the syringe depicted in Figure 4 is of the type prefilled with a medical liquid such as heparin. The syringe depicted in Figure 4 has a pair of resilient pistons 522 spaced apart, with the fluid to be dispensed contained between the pistons. A plunger rod 524 pushes the pistons forward until the forward most piston engages against an entry port 526 which extends in a direction opposite the blunt cannula 518. The forwardmost piston has a frangible portion, which is pierced by the entry port, releasing the liquid contained between the pistons for expulsion through the blunt cannula.

The blunt cannula portion 518 is substantially protected from inadvertent touch contamination by the outer cylindrical wall 516. To permit the blunt cannula to be used, however, with the in-line injection site 492, a pair of opposed, generally U-shaped recesses 528 are provided in the cylindrical wall for receiving the inlet and outlet portions 494, 496 of the in-line injection or sampling site when the cannula is attached to it. This arrangement is depicted in a perspective view in Figure 5. As shown there, the blunt cannula device 512 may be attached to the in-line injection site by inserting the blunt cannula portion into the pre-slit injection or sampling site, with the U-shaped recesses 528 receive the inlet and outlet portions 494, 496 of the in-line injection or sampling site, thus allowing the bare cannula to be inserted sufficiently far into the pre-slit injection or sampling site.

## Claims

1. An in-line injection or sampling site usable with a blunt cannula device, the injection or sampling site comprising a housing having an inlet (494) and an outlet (496) with a fluid channel (498) therebetween, the inlet and outlet being parallel, means defining an access aperture (496a) communicating with the channel and flexible means (502) carried by the housing for sealing the access aperture (496a), the flexible means having a resealable opening therein, such that a blunt cannula can be sealingly inserted through the opening and removed therefrom, so that a cannula inserted in the opening of the flexible means (502) is placed in fluid communication with the channel, characterised in that access aperture (496a) in the housing (500) has a tapered interior surface (501) interacting with the flexible means (502).

2. An in-line injection or sampling site according to Claim 1, wherein said taper is 5° to 20°.

3. An in-line injection or sampling site according to Claim 2, wherein said taper is 12°.

4. An in-line injection or sampling site according to any preceding claim, including swaged end members (504) on the housing (500) forcing the exterior surface of the flexible means (502) into a dome shape.

5. An in-line injection or sampling site according to any preceding claim in combination with a blunt cannula device (458) having a pair of retaining fingers (460) with retention means (468) at their ends, the fingers being biased to engage with a shoulder (508) on the injection site and having grippable parts (470) squeezable together to move the retention means (468) apart to release the cannula device from the injection or sampling site.

## Patentansprüche

1. In-Line-Injektions- oder -Probeentnahmestelle, die mit einer stumpfen Kanüleneinrichtung verwendbar ist, wobei die Injektions- oder -Probeentnahmestelle aufweist: ein Gehäuse, das einen Einlaß (494) und einen Auslaß (496) mit einem Fluidkanal (498) dazwischen hat, wobei der Einlaß und der Auslaß parallel sind, eine Einrichtung, die eine Zugangsöffnung (496a) definiert, die mit dem Kanal in Verbindung steht, und eine flexible Einrichtung (502), die an dem Gehäuse getragen ist, um die Zugangsöffnung (496a) abzudichten, wobei die flexible Einrichtung eine wiederabdichtbare Öffnung darin hat, so daß eine stumpfe Kanüle abdichtend durch die Öffnung eingeführt und daraus entfernt werden kann, so daß eine in die Öffnung der flexiblen Einrichtung (502) eingeführte Kanüle in Fluidverbindung mit dem Kanal gebracht wird, dadurch gekennzeichnet, daß die Zugangsöffnung (496a) in dem Gehäuse (500) eine konisch verjüngte Innenfläche (501) hat, die mit der flexiblen Einrichtung (502) zusammenwirkt.

2. In-Line-Injektions- oder -Probeentnahmestelle nach Anspruch 1, wobei die Konizität 5° bis 20° ist.

3. In-Line-Injektions- oder -Probeentnahmestelle nach Anspruch 2, wobei die Konizität 12° beträgt.

4. In-Line-Injektions- oder -Probeentnahmestelle nach einem der vorhergehenden Ansprüche, die eingezogene Endelemente (504) an dem Gehäuse (500) aufweist, die die Außenfläche der flexiblen Einrichtung (502) in eine Kuppelgestalt drücken.

5. In-Line-Injektions- oder -Probeentnahmestelle nach einem der vorhergehenden Ansprüche in Kombination mit einer stumpfen Kanüleneinrichtung (458), die ein Paar von Festlegefingern (460) mit Haltemitteln (468) an ihren Enden hat, wobei die Finger vorgespannt sind, um mit einer Schulter (508) an der Injektionsstelle in Eingriff zu gelangen, und greifbare Teile (470) haben, die zusammendrückbar sind, um die Haltemittel (468) auseinanderzuspreizen, um die Kanüleneinrichtung von der Injektions- oder -Probeentnahmestelle zu lösen.

## Revendications

1. Site d'injection ou de prélèvement en ligne utilisable avec un dispositif de canule arrondie, le site d'injection ou de prélèvement comprenant un boîtier présentant une entrée (494) et une sortie (496) avec un canal (498) de fluide entre elles, l'entrée et la sortie étant parallèles, des moyens délimitant une ouverture (496a) d'accès communiquant avec le canal et des moyens (502) souples portés par le boîtier pour obturer l'ouverture (496a) d'accès, une ouverture libérable se trouvant à l'intérieur des moyens souples, de sorte qu'une canule arrondie peut être introduite à travers l'ouverture et peut en être ôtée de façon étanche, de sorte qu'une canule introduite dans l'ouverture des moyens (502) souples est placée en communication de fluide avec le canal, caractérisé en ce que l'ouverture (496a) d'accès dans le boîtier (500) présente une surface (501) intérieure conique coopérant avec les moyens (502) souples.

2. Site d'injection ou de prélèvement en ligne selon la revendication 1, dans lequel l'angle dudit cône est de 5° à 20°.

3. Site d'injection ou de prélèvement en ligne selon la revendication 2, dans lequel l'angle dudit cône est de 12°.

4. Site d'injection ou de prélèvement en ligne selon l'une quelconque des rèvendications précédentes, comprenant des éléments (504) d'extrémité sertis sur le boîtier (500) faisant prendre à la surface extérieure des moyens (502) souples une forme de dôme.

5. Site d'injection ou de prélèvement en ligne selon l'une quelconque des revendications précédentes, combiné à un dispositif (458) de canule arrondie comprenant une paire de doigts (460) de retenue présentant des moyens (468) de retenue à leurs extrémités, les doigts étant poussés pour venir en prise avec un épaulement (508) sur le site d'injection et présentant des éléments (470) de préhension pouvant être serrés ensemble afin d'écarter les moyens (468) de retenue pour libérer le dispositif de canule du site d'injection ou de prélèvement.
